(19) Europäisches Patentamt
European Patent Office
Office européen des brevets

(11) Numéro de publication: **0 250 335**
**A1**

## (12) DEMANDE DE BREVET EUROPEEN

(21) Numéro de dépôt: **87401398.0**

(22) Date de dépôt: **19.06.87**

(51) Int. Cl.⁴: **A 61 K 37/54**
**//(A61K37/54,31:195)**

(30) Priorité: **19.06.86 FR 8608874**

(43) Date de publication de la demande:
**23.12.87 Bulletin 87/52**

(84) Etats contractants désignés:
**AT BE CH DE ES FR GB GR IT LI LU NL SE**

(71) Demandeur: **INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur: **Vila, Jorge**
**43 Avenue Rockefeller**
**F-69003 Lyon (FR)**

Thomasset, Nicole
43 rue du Tonkin
F-69100 Villeurbanne (FR)

Epstein, Alberto
403 Avenue du 8 Mai 1945
F-69300 Caluire (FR)

Wild, Fabian
27 rue Guilloud
F-69003 Lyon (FR)

Doré, Jean-François
30 Impasse du Pinay
F-69570 Dardilly (FR)

(74) Mandataire: **Phélip, Bruno et al**
**c/o Cabinet Harlé & Phélip 21, rue de la Rochefoucauld**
**F-75009 Paris (FR)**

(54) **Médicament et composition médicamenteuse pour le traitement des tumeurs ainsi que pour le traitement des maladies infectieuses dues aux virus.**

(57) Ce médicament consiste en l'association d'une enzyme de degradation d'un amino-acide et dudit amino-acide sous forme hydroxamate. Il inhibe de façon totale la multiplication virale (il appartiendrait à la categorie des inducteurs de l'etat cellulaire antiviral), et il montre une activité cytotoxique sur differentes lignées de tumeurs humaines (aussi bien tumeurs solides que lymphomes), la sélectivité d'action (cytolytique et irréversible sur cellules tumorales, et cytostatique réversible sur cellules normales) laissant à penser qu'il utilise une différence métabolique entre cellule tumorale et cellule normale. La composition médicamenteuse correspondante se présente sous la forme d'une poudre lyophilisée, constituée par le mélange des principes actifs, à dissoudre extemporanément dans une solution physiologique, en vue de l'injection.

EP 0 250 335 A1

**Description**

# MEDICAMENT ET COMPOSITION MEDICAMENTEUSE POUR LE TRAITEMENT DES TUMEURS AINSI QUE POUR LE TRAITEMENT DES MALADIES INFECTIEUSES DUES AUX VIRUS

La présente invention se rapporte a un médicament, ainsi qu'à une composition médicamenteuse, permettant, d'une part de traiter les tumeurs, et, d'autre part, de traiter les maladies infectieuses dues aux virus. Ce médicament est constitué par l'association d'une enzyme de dégradation d'un amino-acide et de l'amino-acide correspondant sous sa forme d'hydroxamate . Entrent notamment dans cette définition, les associations médicamenteuses suivantes:

-L-asparaginase et D-aspartique acide β hydroxamate (DAH)
-Phénylalanine ammonialyase et DL-phénylalanine hydroxamate;
-Tryptophanase et DL-tryptophane hydroxamate; et
-Tyrosinase et L-tyrosine hydroxamate.

On exposera ci-après, tout d'abord l'activité antitumorale de l'une de ces associations, à savoir, celle de la L-asparaginase et du D-aspartique acide hydroxamate, et ensuite son activité antivirale.

## I - ACTIVITE ANTITUMORALE DE L'ASSOCIATION L-ASPARAGINASE/D-ASPARTIQUE ACIDE β HYDROXAMATE (DAH)

La découverte de la L-asparaginase comme agent anticancéreux a ouvert la voie pour l'utilisation de métabolites physiologiques dont l'action repose sur la différence biochimique qualitative entre cellule tumorale et cellule normale. Cela lui confère une sélectivité pour certaines cellules tumorales. Bien que seules les néoplasies lymphoïdes répondent au traitement à l'asparaginase de façon reproductible, d'autres tumeurs telles que mélanomes ou adénocarcinomes sont sensibles à l'action de la L-asparaginase. La L-asparaginase catalyse principalement l'hydrolyse de la L-asparaginase en acide aspartique et en ammoniaque. La L-asparagine n'est pas considérée comme un acide aminé essentiel pour les tissus normaux de mammifères. Cet acide aminé est synthétisé par l'asparagine synthétase à partir de l'acide L-aspartique et d'un groupment $NH_3$. Des différents travaux, il ressort que l'activité asparagine synthétase est faible dans les cellules tumorales, bien qu'elles aient un besoin important en asparagine et acide aspartique. Cette dépendance de l'apport extérieur en asparagine des cellules tumorales explique en partie l'effet cytotoxique de la L-asparaginase pour ces cellules.

Les premiers travaux ont été obtenus en 1953 par Kidd qui utilisait le sérum de cobaye pour inhiber la croissance de lymphosarcome de Gardner (6C3HE-D) sur la souris C3H. En 1961, Broome JD. J. Exp. Med. 118 : 121-148, 1963, démontre que l'agent antitumoral du sérum de cobaye est la L-asparaginase. En 1963, Mashburn L.T. et Wriston J.C. Arch. Biochem. Biophys. 105 : 450-452, 1964, mettent en évidence l'activité inhibitrice de la L-asparaginase de Escherichia coli sur la croissance du lymphosarcome 6C3HE-D. Depuis lors, l'asparaginase de nombreuses sources a été purifiée. La plus grande majorité des travaux ont été réalisés avec la L-asparaginase purifiée d'Escherichia coli

### Activité antitumorale de la L-asparaginase sur modèles animaux

L'activité de la L-asparaginase a été démontrée sur plus de 50 tumeurs murines, 3 tumeurs de rat et sur les lymphosarcomes du chien. La majorité des tumeurs sensibles à la L-asparaginase sont d'origine lymphoïde, d'étiologie cependant très diverses (irradiation, uréthanne, DMBA, virus). Parmi les différentes tumeurs greffées de la souris consanguine, utilisées pour l'évaluation préclinique des agents anticancéreux, la L-asparaginase a une activité seulement sur les tumeurs très sensibles aux agents cytostatiques, telles que les leucémies P388, carcinosarcome de Walker 256. Sur les modèles tumoraux, il a été fermement établi que les tumeurs rentrent en compétition avec l'hôte pour retenir les composés nitrogènes (Corrascosa JD et al., Cancer Res. 44 : 3831-3835, 1984). Parmi ces composés, Corrascosa a montré, sur des souris inoculées avec les cellules tumorales de Erhlish, l'existence d'un transfert très important de glutamine et asparagine, du plasma et du liquide ascitique de l'hôte via la tumeur. Les travaux de Root et Broome (Broome J D et Schwartz J H. Biochem. Biophys. Acta 138 : 637-639, 1967) montrent que les tumeurs sensibles à la L-asparaginase ont une activité faible ou nulle d'asparagine synthétase. En plus, les cellules lymphoïdes qui échappent à l'action de la L-asparaginase acquièrent une activité L-asparaginase synthétase importante (Horowitz B, Madras BK, Meister D. et al. Science 160 : 533-535, 1968). De ces travaux, il ressort que l'asparagine, synthétisée par les cellules tumorales ou venant de l'hôte, joue un rôle clé dans la croissance des cellules tumorales.

Adamson R.H. et Fabro S. Cancer Chemother. Repond. 52 : 617-626, 1968, ont étudié la relation dose-réponse de la L-asparaginase. Une dose importante de 1000 UI/kg produit la guérison de tous les animaux porteurs de leucémie L5184; cependant, l'injection de L-asparaginase en doses fractionnées sur plusieurs jours ne montre pas d'effet sur les animaux. Dowley W.C, Corwet J. Henson D. et al. Proc. Soc. Exp. Biol. Med. 123 : 133-137, 1966 montrent que des doses suffisantes d'asparaginase peuvent inhiber le développement d'une tumeur, quel que soit son mode d'inoculation chez l'animal : sous-cutané, intrapéritonéal, ou intracérébral. Compte-tenu que la L-asparaginase a un poids moléculaire de 139 000 et ne traverse pas de façon importante la barrière hématocéphalique, on peut conclure que l'effet cytotoxique de la L-asparaginase se fait, soit par déplétion en asparagine du milieu sanguin suivie de sa déplétion dans le milieu céphalorachidien, soit par la présence d'un composé résultant de l'activité enzymatique dans le milieu sanguin et qui peut traverser la barrière hématoencéphalique.

## Efficacité de la L-asparaginase chez l'homme

La thérapie des maladies malignes avec la L-asparaginase présente une efficacité limitée pour différentes raisons. La grande majorité des néoplasies peuvent synthétiser la L-ASN et ne sont pas dépendantes de l'asparaginase du milieu.

Comme il a été montré (Wood J S et Handschumacher R E. Ann. Physiol. 221 : 2940, 1974), les cellules lymphoïdes qui échappent à l'action de l'asparaginase acquièrent une activité L-asparaginase synthétase importante. Cette augmentation d'activité se fait :
- soit par dérépression dans la synthèse de l'enzyme. Arfin et al., Proc. Natl. Acad. Sci. USA 78 : 5724-5728, 1981, ont montré que, dans des cellules d'ovaires de hamster chinois (CHO), cultivées en milieu déprimé en aspargine, l'augmentation d'activité ASN-synthétase s'accompagne d'une diminution de l'activité asparaginyl tRNA synthétase ;
- soit par amplification du gène de l'enzyme.

La déplétion en asparagine du milieu nécessite l'utilisation, d'une part, de la L-asparaginase et, d'autre part, d'inhibiteurs de la L-asparagine synthétase. On pouvait ainsi espérer que des inhibiteurs de la biosynthèse de l'asparagine augmenteraient l'activité de l'asparaginase. Urew J.R. et al., Biochem. Pharmacol., 26 : 1405-1410 ont étudié l'activité de divers inhibiteurs de l'asparagine synthétase dont le mélange L-asparaginase avec le DL β-aspartique acide hydroxamate et une autre molécule qui s'est révélée plus active que celui-ci dans les essais préliminaires, le β-aspartylméthylamide. Si ce dernier s'est révélé actif sur des tumeurs lymphoblastiques de la souris dont certaines sont résistantes à l'asparaginase, l'association L-β-aspartylméthylamide/L-asparaginase n'a pas présenté l'activité attendue : aucune potentialisation de l'activité de l'un par rapport à l'autre n'a été observée ; en revanche, l'activité de l'asparaginase était diminuée en présence de cet inhibiteur.

Sur ces bases théoriques, a été étudié -ce qui a permis de conduire à la présente invention- l'effet d'une molécule de D-aspartique acide β hydroxamate associée à la L-asparaginase, dans l'espoir de pouvoir accroître l'effet cytolytique de cette dernière sur des tumeurs sensibles, de contrôler le mécanisme biologique et génétique de résistance à celle-ci, et d'élargir, si possible, le spectre de la L-asparaginase jusqu'aux tumeurs insensibles .

L'action de cette association DAH/L-asaparaginase sur pluseurs types de cellules tumorales et normales a donc été étudiée aussi bien in vitro que in vivo.

## A - ETUDE EN CULTURE CELLULAIRE

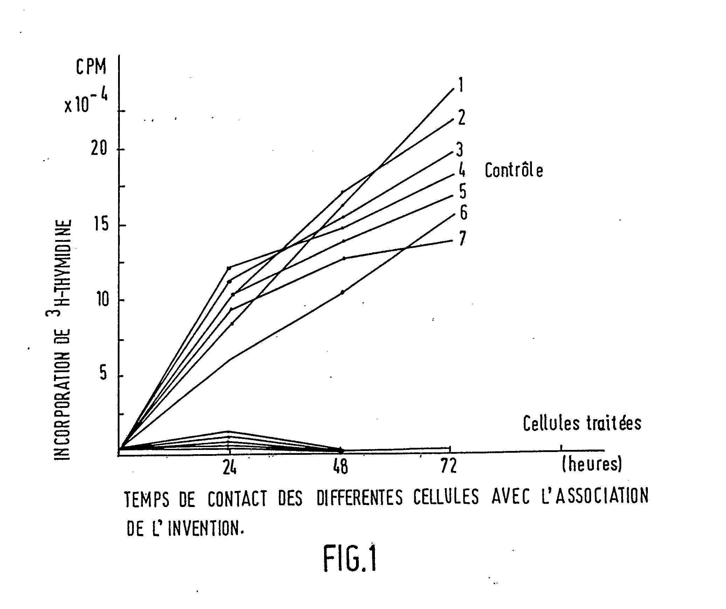

L'effet de l'association précitée a été étudié in vitro sur diverses lignées cellulaires obtenues à partir de tumeurs humaines et fibroblastes de poumon foetal humain diploïde (MRC5). Parmi les tumeurs humaines, on a observé un arrêt de la synthèse de l'ADN, aussi bien sur les tumeurs solides : carcinome de tumeur mammaire (lignée MCF7), lignée de tumeur d'Ewing, mélanome, carcinome du colon (lignée HT29), que sur les lymphomes : cellules de tumeurs de Burkitt, cellules du lymphome murin RBL5 ou de leucémie L1210 (Figure 1).

Légende de la figure 1 :
- en abscisses: Temps de contact de différentes cellules avec l'association L-asparaginase/DAH ;
- en ordonnées : Incorporation de $^3$H-thymidine.
1 - MCF 7 ; 2 - RBL 5 ; 3 - BL60 ; 4 - L1210 ; 5 - HT29 ; 6 - NTerDau ; 7 - Ewing.

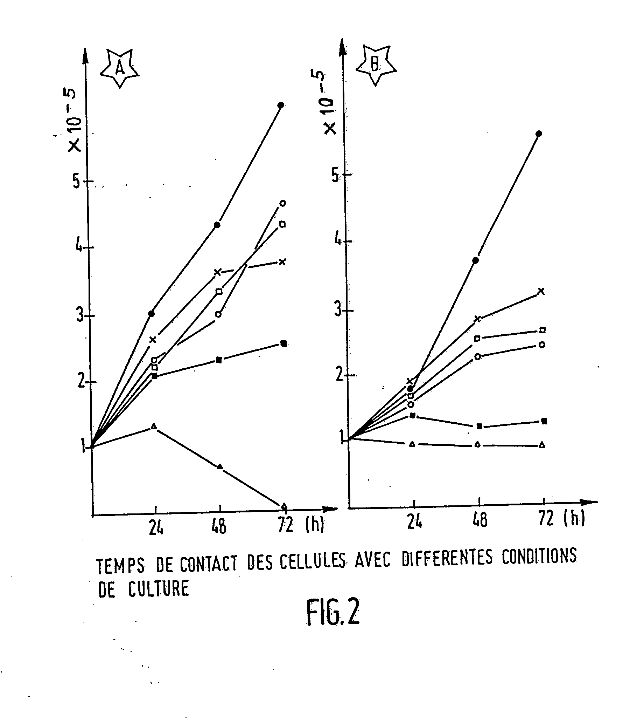

En prenant les cellules de mélanome comme exemple, on a observé une faible inhibition de la croissance dans un milieu dépourvu en asparagine. Cette faible inhibition pouvait être en relation avec la capacité pour ces cellules de synthétiser la L-asparagine par la L-asparagine synthétase, comme il a été démontré par Horowitz et al (Science 160:533 1968). Ces cellules devraient donc être résistantes à l'action de l'asparaginase. L'effet de résistance à l'asparaginase est en fait dépendante du type cellulaire et de la dose utilisée. On voit, pour la dose de 0,4 U/ml d'asparaginase, une inhibition à 72 heures de 40% de la croissance des cellules de mélanome et de 42% pour les cellules normales MRC5. L'inhibition de croissance obtenue lorsque l'on associe l'asparaginase et le DAH dans le milieu de culture des cellules normales est du même ordre que celle obtenue dans un milieu déprimé en asparagine, auquel on ajoute l'asparaginase. En revanche, pour les cellules tumorales, une potentialisation de l'effet inhibiteur est observée lorsque la L-asparaginase est ajoutée au DAH dans un milieu complet par rapport à la croissance observée dans un milieu dépourvu en asparagine + DAH (Figure 2).

Légende de la figure 2 :
- en abscisses : Temps de contact (heures) des cellules avec différentes conditions de culture.
- A - cellules de mélanome NterDau ;
- B - cellules normales MRC5.
- en ordonnées : nombre de cellules vivantes
● milieu normal contrôle ; × milieu plus L-asparaginase 0,4 U/ml ; O milieu plus DAH 400 µM ; □ milieu déprimé en asparagine ; ■ milieu déprimé en asparagine plus DAH 400 µM ; Δ milieu avec L-asparaginase 0,4 U/ml plus DAH 400 µM.

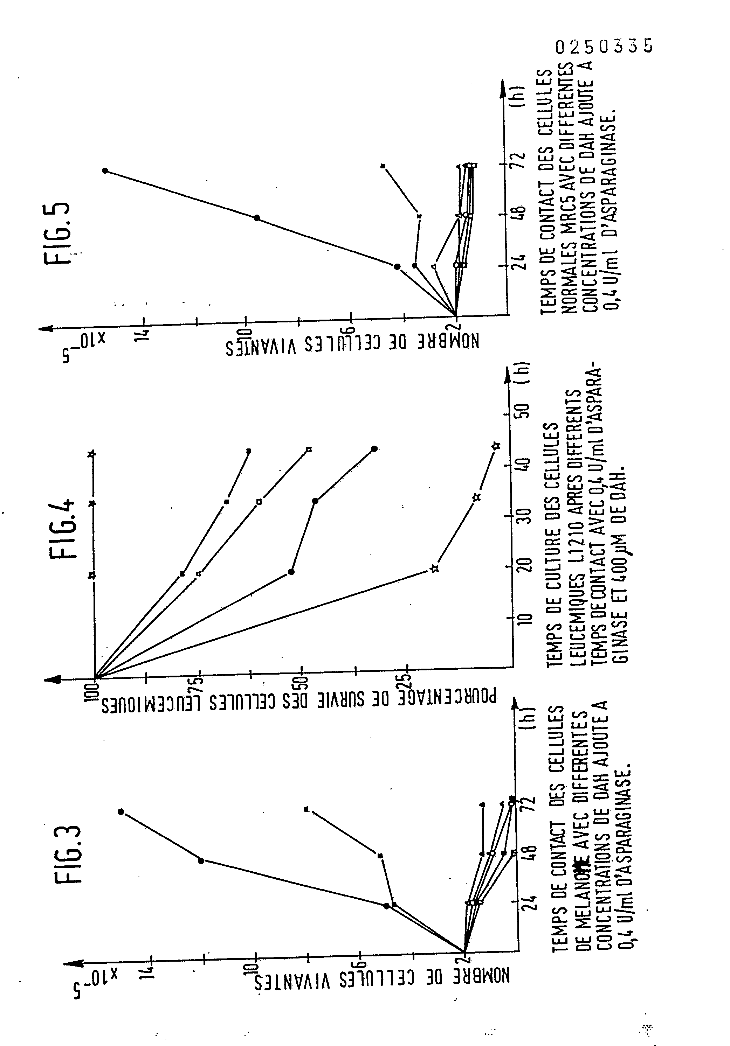

L'effet inhibiteur total de la croissance des cellules tumorales est dépendant de la dose (Figure 3) et du temps de contact avec les cellules. Dès 2 heures de contact de l'association avec les cellules leucémiques L1210, on observe une mort cellulaire qui s'accroît au cours de la culture des cellules (Figure 4). Un contact de 20 heures aboutit à une mort cellulaire totale pour ces cultures.

en revanche, l'association asparaginase/DAH provoque un arrêt de la synthèse de l'ADN des cellules normales MRC5, sans que cela s'accompagne de mort cellulaire pour des doses allant jusqu'à 2mM (Figure 5). On observe un effet cytostatique sur les cellules normales qui est réversible après élimination de la drogue.

Légende de la figure 3 :
- en absicisses : Temps de contact des cellules de mélanome avec différentes concentrations de D-aspartyl hydroxamate (DAH) ajouté à o,4 U/ml d'aparaginase :
● Témoin ; cellules de mélanome cultivées avec 0,4
U/ml d'aparaginase plus D-aspartyl hydroxamate :
■ 100 μM ; Δ 200 μM ; ▲ 400 μM ; O 800 μM ;
🌑1mM ; □ 2 mM
- en ordonnées : nombre de cellules vivantes.

Légende de la figure 4 :
- en abscisses : Temps de culture des cellules
leucémiques L1210 après différents de contact avec
0,4 U/ml asparaginase et 400 μM aspartyl hydroxamate : ✡Témoin L1210 ; Cellules 1210 incubées
avec l'association pendant : ■ 2 heures, □ 6
heures, ● 8 heures,✿20 heures ;
- en ordonnées : pourcentage de survie des cellules
leucémiques L1210.

Légende le la figure 5 :
- en abscisses : Temps de contact des cellules
normales MRC5 avec différentes concentrations de
D-aspartyl hydroxamate ajouté à 0,4 U/ml d'asparaginase : ● témoin ; cellules MRC5 cultivées avec 0,4
U/ml d'asparaginase plus β aspartyl hydroxamate ■
100 μM ; Δ 200 μM ; ▲ 400 μM ; O 800μM;✿1 nM ;
□ 2 mM ;
- en ordonnées nombre de cellules vivantes.

Il a, par ailleurs été observé que les autres
associations précitées provoquent le même effet
cytolytique sur cellules tumorales et cytostatiques
réversible sur cellules normales.

## B - ETUDES IN VIVO SUR DES MODELES MURINS

compte tenu des résultats in vitro, l'activité
antitumorale de cette association médicamenteuse
a été étudiée sur trois modèles tumoraux murins :
L1210, RBL5, B16. Les cellules leucémiques L1210
sont maintenues par passages intrapéritonéaux
dans la souris DBA/2. Le nombre de cellules
utilisées pour les essais thérapeutiques est de $5.10^5$
cellules par souris.

Les cellules de lymphome murin RBL5 sont
maintenues par passages intrapéritonéaux successifs chez la souris C57b16 et injectées, pour les
essais, à la dose de $10^5$ cellules par souris. Les
cellules de mélanome murin B16 sont maintenues
par passages subcutanés chez la souris C57b16 et
injectées intrapéritonéalement à $10^6$ pour les essais
L'activité antitumorale expérimentale des drogues a
été évaluée en comparant les durées de survie des
animaux auxquels sont injectées les cellules tumorales avec ou sans drogue. Une augmentation de
30% de la durée de survie des souris traitées par
une drogue par rapport aux témoins non traités est
considérée comme significative. Dans le cas de
traitement par une association de drogues, il y a
synergie pharmacologique lorsque l'augmentation
de la survie des souris représente plus de 40% de la
réponse obtenue par chacun des produits.

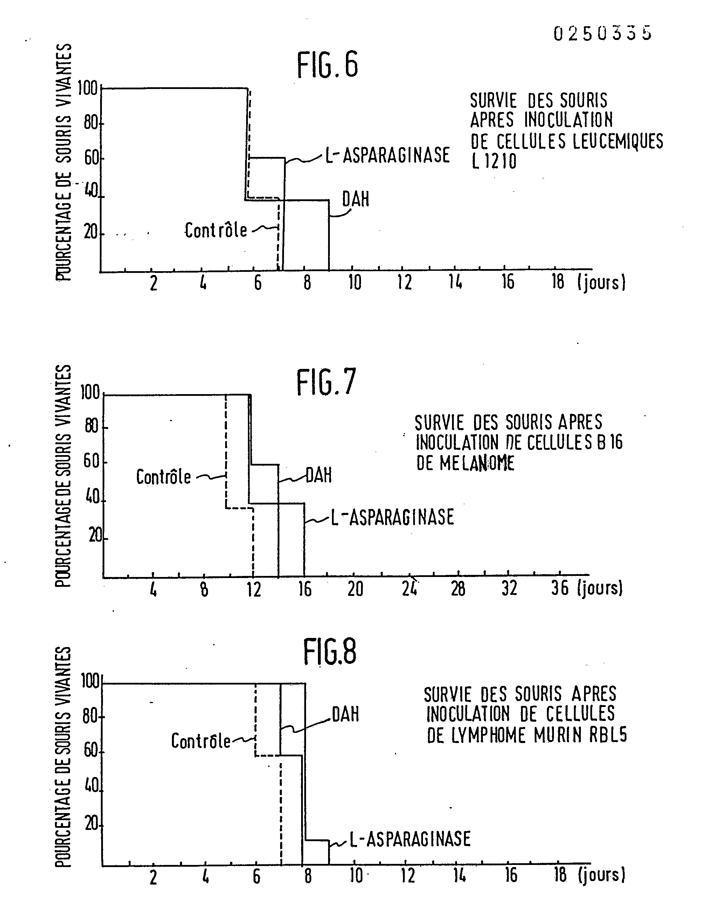

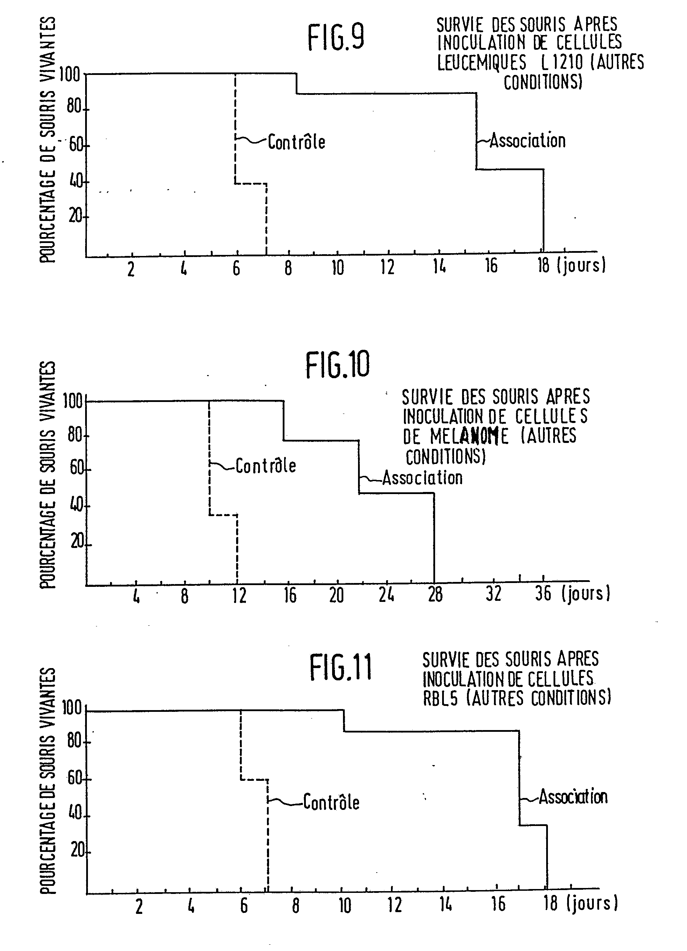

Les trois modèles murins étudiés montrent une
résistance au traitement avec la L-asparaginase
seule ou du DAH seul (Figures 6-7-8). En revanche,
le traitement avec l'association L-asparaginase-DAH,
dans les conditions qui ont été définies, met en
évidence une synergie pharmacologique avec une augmentation de la survie des souris ainsi traitées
de plus de 120% (courbes 9-10-11)

Légendes des figures 6 à 11 :

1°) - en abscisses : survie des souris (jours)

- Figure 6 :
Courbe de survie des souris DBA/2-$B_6D_2F_1$
inoculées avec les cellules leucémiques L1210
($5x10^5$ cellules dans 0,5 ml IP), après traitement avec
:
- L-asparaginase : 200 UI/kg injectée 2 fois par jour
pendant 5 jours ;
- DAH : 600 mg/kg( dose pendant 5 jours);

- Figure 7 :
Courbe de survie des souris C57b16 inoculées
avec les cellules B16 de mélanome ($10^6$ cellules
dans 0,5 ml IP) après traitement avec :
- L-asparaginase : 200 UI/kg 2 fois par jour pendant 5
jours ;
- DAH : 600 mg/kg (dose pendant 5 jours);

- Figure 8 :
Courbe de survie des souris C57b16 inoculées
avec les cellules de lymphome murin RBL5 ($10^6$
cellules dans 0,5ml IP) après traitement avec :
- L-asparaginase : 200 UI/kg 2 fois par jour pendant 5
jours ;
- DAH : 600 mg/kg pendant 5 jours ;

- figure 9 :
Courbe de survie des souris DBA/2 inoculées
avec les cellules L1210 ($5m10^5$ cellules dans 0,5 ml)
après traitement avec :
- L-asparaginase et DAH, administrés aux jours
1,2,3 - 7,8,9 - 14,15,16, aux doses suivantes:
L-asparaginase : 200 UI/kg
et DAH : 600 mg/kg (dose/jour);

- Figure 10 :
Courbe de survie des souris C57b16 inoculées
avec les cellules de mélanome B16 ($10^6$ cellules
dans 0,5 ml) après traitement avec l'association
L-asparaginase et le DAH, administrés aux jours
1,2,3 - 7,8,9 - 15,16,17 aux doses suivantes :
- L-asparaginase : 200 UI/kg/jour,
- DAH : 600 mg/kg (dose/jour);

- Figure 11 :
Courbe de survie des souris C57b16 inoculées
avec les cellules RBL5 ($10^6$ cellules dans 0,5 ml)
après traitement avec la L-asparaginase et le DAH,
administrés aux jours 1,2,3 - 7,8,9 - 14,15, aux doses
suivantes :
- L-asparaginase : 200 UI/kg/jour
- DAH : 600 mg/kg (dose/jour);

2°) en ordonnées : pourcentage de souris vivantes.
Les groupes traités et non traités contiennent 8
souris.

Des résultats ci-dessus, il ressort que l'association L-asparaginase/DAH est capable d'inhiber sélectivement la croissance de cellules tumorales aussi variées que tumeur du sein, du colon, mélanome et lymphome, alors qu'elle provoque un arrêt du cycle cellulaire de la cellule normale. L'addition simultanée des deux composés est indispensable aussi bien pour leur effet cytolytique sur les cellules tumorales que cytostatique sur cellules normales. L'asparaginase présente un rôle particulier qui ne se limite pas pour des cellules tumorales à la déplétion en ASN du milieu.

L'effet cytolytique de l'association est, dans certaines conditions, irréversible sur les cellules tumorales alors qu'il est réversible sur les cellules normales.

L'activité antitumorale de l'association L-asparaginase et DAH, étudiée sur trois modèles murins, montre une synergie pharmacologique avec une augmentation de la survie des souris traitées de plus de 120%.

## II - ACTIVITE ANTIVIRALE DE L'ASSOCIATION L-ASPARAGINASE/D-ASPARTIQUE ACIDE β HYDROXAMATE (DAH)

Bien que des progrès aient été accomplis en ce qui concerne la lutte contre les infections virales, on ne dispose pas encore d'une gamme d'agents antiviraux aussi étendue, sure et efficace que le sont, par exemple, les antibiotiques.

De par leur mode d'action, les agents antiviraux peuvent être classés, d'une façon large, en trois catégories, les frontières entre celles-ci étant souvent arbitraires :

- la première catégorie, à savoir celle des agents chimiques, constitue le groupe le plus étendu et celui qui se développe le plus vite. Etant donné que les virus sont des parasites intracellulaires obligés, l'efficacité de ces agents est déterminée par leur capacité à différencier le métabolisme viral du métabolisme cellulaire, autrement dit le métabolisme de la cellule infectée et celui de la cellule non infectée. En d'autres termes, la caractéristique essentielle de ce groupe d'agents est leur spécificité d'action. Celle-ci détermine en même temps la force et la faiblesse de ces agents, car, s'ils sont souvent très efficaces, du fait qu'ils peuvent inhiber spécifiquement une enzyme virale, leur spectre d'action est généralement très limité et ils finissent souvent par sélectionner des variants viraux résistants, de la même manière que le font les antibiotiques avec les bactéries (De Clercq E., Biochem. J.202 : 1-13, 1982; Becker Y. et Hadar J., Prog. Med. Virol. 26:1-44, 1980);
- la deuxième catégorie est constituée par les stimulateurs du système immun. Mis à part les différents types de vaccins, ce groupe est constitué par des agents capables d'activer les défenses non spécifiques (macrophages, cellules "tueuses" naturelles) ou plus rarement, les défenses spécifiques (lymphocytes "B" ou "T") de l'organisme. Pour la plupart, ces agents sont encore en phase expérimentale. Contrairement aux agents chimiques, les caractéristiques essentielles de leur mode d'action sont leur spectre d'action très large et le fait qu'ils ne peuvent pas sélectionner de variants résistants. Cependant, ils sont, pour l'instant, en nombre limité et peu efficaces, bien que des progrès aient été récemment accomplis dans ce sens (Koff W.C.,Showalter S.D., Hampar B. et Fidler I. J., Science 228: 495-496, 1985).

- la troisième catégorie est celle des inducteurs de l'état cellulaire antiviral, groupe qui comprend l'interféron (IFN), les inducteurs d'IFN et les substituts d'IFN. Il s'agit d'un groupe encore très réduit de substances qui se caractérisent comme éveillant dans les cellules un état de résistance à l'infection virale. Ces agents cumulent la puissance de l'action au niveau cellulaire des agents chimiques et le spectre d'action très large des activateurs du système immun. Du fait qu'ils agissent sur toutes les cellules, qu'elles aient été infectées ou non, un élément déterminant de leur efficacité est leur faible pouvoir toxique envers les cellules.

Il a maintenant été découvert que l'association du D-aspartique acide β hydroxamate et de la L-asparaginase inhibe de façon totale la multiplication virale.

On peut penser que cette association de substances appartient à la troisième catégorie sus-indiquée des agents antiviraux, bien que son mode précis d'action n'ait pas encore été totalement élucidé.

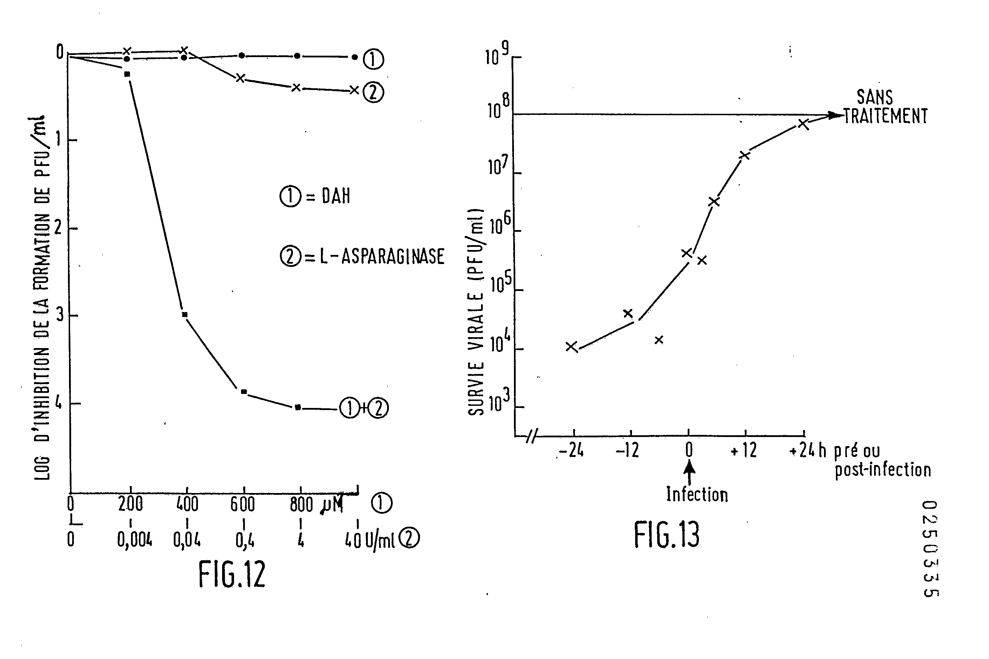

1°/ Une expérience in vitro a permis de mettre en évidence que l'association D-aspartique acide β hydroxamate (DAH)/L-asparaginase provoque une inhibition très significative de deux virus pourtant très éloignés à plusieurs égards, l'un étant un virus à ARN (rougeole) et l'autre, un virus à ADN (HSV.1) :
Dés cellules VERO (lignée de cellules "normales") ont été infectées par le virus de l'herpès simplex du type 1 (HSV.1) ou par le virus de la rougeole à une multiplicité d'infection d'une particule infectieuse par cellule. Les cellules infectées ont été traitées par DAH/L-asparaginase aux doses indiquées sur la figure 12 . l'inhibition observée, (exprimée en logarithme d'inhibition de la formation de plages de lyse cellulaire par le virus (PFU-"plage forming unit") par ml de milieu), semble apparaître brusquement après avoir dépassé une concentration "seuil" située autour de 400μM de DAH en présence de 0,4U/ml de l'enzyme L-asparaginase.

Le fait que la concentration optimale soit la même pour les deux virus testés, qui comme indiqué ci-dessus, sont très différents, est en faveur d'une action de l'association au niveau cellulaire, action capable d'éveiller un état de résistance envers les virus infectants.

2°/ Des expériences ont été menées pour déterminer l'effet du temps de prétraitement, ou du traitement post-infection avec cette association DAH/L-asparaginase :
Les cellules VERO infectées pr l'HSU.1 à une multiplicité de 1 PFU/ml, ont été prétraitées ou post-traitées par l'association DAH/L-asparginase aux doses respectives de 600 μM et de 0,4 U/ml de L-asparaginase. La production virale a été comparée avec un témoin traité. Les resultats sont indiqués sur la figure 13.

On constate que le prétraitement (entre -24 heures et 0) provoque une inhibition de la multiplication qui atteint plus de 4 logs. à -24 heures et près de 3 logs à -6 heures. après l'infection, l'effet inhibiteur est significatif jusqu'à environ 6 heures (près de 2 logs) et diminue par la suite.

3°/ D'autres expériences, qui ont été effectuées, montrent que l'inhibition observée est indépendante de la multiplicité d'infection, au moins jusqu'à une multiplicité de dix particules infectieuses par cellule.

Le niveau d'inhibition maximal observé est égal, sinon supérieur, à celui obtenu avec les meilleurs inhibiteurs de la multiplication virale dont on dispose aujourd'hui. Le faible taux, voire l'absence de toxicité de l'association DAH/L-asparaginase pour les cellules (infectées ou non), mérite également d'être souligné.

Il convient également de préciser que chacun des composants de cette association considéré isolément ne présente aucun effet antiviral (Goswani B.B., Gosselin H., Kubach J.L. et Sarma O.K. Virology 137 : 400-407, 1984).

La présente invention a également pour objet une nouvelle composition médicamenteuse, synergique, destinée notamment traitement des tumeurs, et au traitement des maladies infectieuses dues aux virus, caractérisée par le fait qu'elle comprend, à titre d'agents actifs, d'une part, une enzyme de dégradation d'un amino-acide et, d'autre part, ledit amino-acide sous formes hydroxamate, présentés simultanément ou séparément. Des exemples de telles associations de principes actifs sont indiqués ci-dessus.

Chaque principe actif se présente sous la forme d'une poudre lyophilisée à dissoudre extemporanément dans une solution physiologique en vue de l'injection, soit simultanée, soit séparée, des deux principes actifs. L'administration s'effectue par voie parentérale (intraveineuse, intrapéritonéale, dans le liquide céphalorachidien, etc.)

Les concentrations respectives des principes actifs dans les solutions d'injection sont les suivantes :
L-asparaginase : 200 IU/l
DAH : 0,6 à 1,5 g/l

Par ailleurs, la composition de l'invention peut également contenir des additifs inertes ou pharmacodynamiquement actifs.

La présente invention conerne aussi l'utilisation d'une enzyme de dégradation d'un amino-acide et dudit amino-acide sous forme hydroxamate, pour fabriquer une composition médicamenteuse destinée au traitement des tumeurs, ainsi que l'utilisation d'une enzyme de dégradation d'un amino-acide et dudit amino-acide sous forme hydroxamate, pour fabriquer une composition médicamenteuse destinée au traitement des maladies infectieuses dues aux virus.

Enfin, la présente invention concerne un procédé de préparation d'une composition pharmaceutique destinée au traitement des tumeurs, caractérisé par le fait qu'on prépare d'une part, une enzyme de dégradation d'un amino-acide, et, d'autre part, ledit amino-acide sous forme hydroxamate, sous une forme de poudre lyophilisée destinée à être dissoute extemporanément dans une solution physiologique, en vue de l'injection, ainsi qu'un procédé de préparation d'une composition pharmaceutique destinée au traitement des maladies infectieuses dues aux virus, caractérisé par le fait qu'on prépare d'une part, une enzyme de dégradation d'un amino-acide, et, d'autre part, ledit amino-acide sous forme hydroxamate, sous une forme de poudre lyophilisée destinée à être dissoute extemporanément dans une solution physiologique, en vue de l'injection.

**Revendications**

1 - Médicament, caractérisé par le fait qu'il consiste en l'association d'une enzyme de dégradation d'un amino-acide et dudit amino-acide sous forme hydroxamate.

2 - Médicament selon la revendication 1, caractérisé par le fait qu'il consiste en l'association de la L-asparaginase et du D-aspartique acide β hydroxamate (DAH)

3 - Médicament selon la revendication 1, caractérisé par le fait qu'il consiste en l'association de la phénylalanine ammonialyase et du DL-phénylalanine hydroxamate.

4 - Médicament selon la revendication 1, caractérisé par le fait qu'il consiste en l'association de la tryptophanase et du DL-tryptophane hydroxamate.

5 - Médicament selon la revendication 1, caractérisé par le fait qu'il consiste en l'association de la tyrosinase et du L-tyrosine hydroxamate.

6 - Médicament selon l'une des revendications 1 à 5, caractérisé par le fait qu'il est destiné au traitement des tumeurs.

7 - Médicament selon l'une des revendications 1 à 5, caractérisé par le fait qu'il est destiné au traitement des maladies infectieuses dues aux virus.

8 - Composition médicamenteuse synergique, destinée notamment au traitement des tumeurs et au traitement des maladies infectieuses dues aux virus, caractérisée par le fait qu'elle comprend, à titre d'agents actifs, d'une part, une enzyme de dégradation d'un amino-acide, et, d'autre part, ledit amino-acide sous forme hydroxamate, présentés séparément ou simultanément.

9 - Composition médicamenteuse selon la revendication 8, caractérisée par le fait que chaque principe actif se présente sous la forme d'une poudre lyophilisée à dissoudre extemporanément dans une solution physiologique en vue de l'injection, soit simultanée, soit séparée.

10 - Composition selon l'une des revendications 8 et 9, caractérisée par le fait que les concentrations respectives des principes actifs dans les solutions d'injection sont les suivantes :
L-asparaginase : 200 IU/l
D A H : 0,6 à 1,5 g/l

11 - Composition selon l'une des revendications 8 à 10, caractérisée par le fait qu'elle contient des additifs inertes ou pharmacodynamiquement actifs.

12 - Utilisation d'une enzyme de dégradation d'un amino-acide et dudit amino-acide sous forme hydroxamate, pour fabriquer une composition médicamenteuse destinée au traitement des tumeurs.

13 - Utilisation d'une enzyme de dégradation d'un amino-acide et dudit amino-acide sous forme hydroxamate, pour fabriquer une composition médicamenteuse destinée au traitement des maladies infectieuses dues aux virus.

14 - Procédé de préparation d'une composition pharmaceutique destinée au traitement des tumeurs, caractérisé par le fait qu'on prépare d'une part, une enzyme de dégradation d'un amino-acide, et, d'autre part, ledit amino-acide sous forme hydroxamate, sous une forme de poudre lyophilisée destinée à être dissoute extemporanément dans une solution physiologique, en vue de l'injection.

15 - Procédé de préparation d'une composition pharmaceutique destinée au traitement des maladies infectieuses dues aux virus, caractérisé par le fait qu'on prépare d'une part, une enzyme de dégradation d'un amino-acide, et, d'autre part, ledit amino-acide sous forme hydroxamate, sous une forme de poudre lyophilisée destinée à être dissoute extemporanément dans une solution physiologique, en vue de l'injection.

TEMPS DE CONTACT DES DIFFERENTES CELLULES AVEC L'ASSOCIATION DE L'INVENTION.

FIG.1

TEMPS DE CONTACT DES CELLULES AVEC DIFFERENTES CONDITIONS DE CULTURE

FIG.2

0250335

FIG.3

NOMBRE DE CELLULES VIVANTES

x10⁻⁵

TEMPS DE CONTACT DES CELLULES
DE MELANOME AVEC DIFFERENTES
CONCENTRATIONS DE DAH AJOUTE A
0,4 U/ml D'ASPARAGINASE.

FIG.4

POURCENTAGE DE SURVIE DES CELLULES LEUCEMIQUES

TEMPS DE CULTURE DES CELLULES
LEUCEMIQUES L1210 APRES DIFFERENTS
TEMPS DE CONTACT AVEC 0,4 U/ml D'ASPARA-
GINASE ET 400 μM DE DAH.

FIG.5

NOMBRE DE CELLULES VIVANTES

x10⁻⁵

TEMPS DE CONTACT DES CELLULES
NORMALES MRC5 AVEC DIFFERENTES
CONCENTRATIONS DE DAH AJOUTE A
0,4 U/ml D'ASPARAGINASE.

0250335

# FIG.6

SURVIE DES SOURIS
APRES INOCULATION
DE CELLULES LEUCEMIQUES
L 1210

# FIG.7

SURVIE DES SOURIS APRES
INOCULATION DE CELLULES B 16
DE MELANOME

# FIG.8

SURVIE DES SOURIS APRES
INOCULATION DE CELLULES
DE LYMPHOME MURIN RBL5

0250335

FIG.9

SURVIE DES SOURIS APRES INOCULATION DE CELLULES LEUCEMIQUES L1210 (AUTRES CONDITIONS)

FIG.10

SURVIE DES SOURIS APRES INOCULATION DE CELLULES DE MELANOME (AUTRES CONDITIONS)

FIG.11

SURVIE DES SOURIS APRES INOCULATION DE CELLULES RBL5 (AUTRES CONDITIONS)

FIG.12

FIG.13

① = DAH

② = L-ASPARAGINASE

LOG D'INHIBITION DE LA FORMATION DE PFU/ml

SURVIE VIRALE (PFU/ml)

SANS TRAITEMENT

Infection

+24 h pré ou post-infection

0250335

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 76, no. 15, 10 avril 1972, page 31, colonne 2 - page 32, colonne 1, abrégé no. 81228z, Columbus, Ohio, US; L. MASHBURN: "Combination chemotherapy with l-asparaginase (EC-2) using P1798 lymphosarcoma", & Cancer 1971, 28(5) 1321-8 <br> * Abrégé * | 1-15 | A 61 K 37/54 // (A 61 K 37/54 A 61 K 31:195) |
| A | CHEMICAL ABSTRACTS, vol. 91, no. 11, 10 septembre 1979, page 59, colonne 1, abrégé no. 83249r, Columbus, Ohio, US; J.R. UREN et al.: "Improvement in the therapeutic, immunological, and clearance properties of Escherichia coli and Erwinia carotovora l-asparaginases by attachment of poly-DL-alanyl peptides", & Cancer res. 1979, 39(6, Pt.1) 1927-33 <br> * Abrégé * | 1-15 | |

DOMAINES TECHNIQUES
RECHERCHES (Int. Cl.4)

A 61 K

Le présent rapport de recherche a été établi pour toutes les revendications

| Lieu de la recherche | Date d'achèvement de la recherche | Examinateur |
|---|---|---|
| LA HAYE | 29-09-1987 | BRINKMANN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03.82